# EUROPEAN PATENT APPLICATION

(11) **EP 4 567 106 A1**
(43) Date of publication of application: **11.06.2025**
(21) Application number: 23853058.8
(22) Date of filing: 11.08.2023
(51) Int. Cl.: C12N 9/04, C12N 15/77, C12P 13/08

(54) **KETOL-ACID REDUCTOISOMERASE VARIANT, AND METHOD FOR PRODUCING L-VALINE BY USING SAME**

(30) Priority: 12.08.2022 KR 20220101231
(71) Applicant: CJ CheilJedang Corporation, Seoul 04560 (KR)
(72) Inventor: OH, Haena, Seoul 04560 (KR); KIM, Seon Hye, Seoul 04560 (KR); YUN, Hyojin, Seoul 04560 (KR); LEE, Hyein, Seoul 04560 (KR)
(74) Representative: Meissner Bolte Partnerschaft mbB
(86) International application number: PCT/KR2023/011906
(87) International publication number: WO 2024/035191

(57) **Abstract**

The present disclosure relates to a ketol-acid reductoisomerase variant, a polynucleotide encoding the variant of the present disclosure, a microorganism producing L-valine comprising the ketol-acid reductoisomerase variant of the present disclosure or a polynucleotide encoding the same, and a method for producing L-valine using the microorganism of the present disclosure.

## Description

### [Technical Field]

The present disclosure relates to a ketol-acid reductoisomerase variant, a polynucleotide encoding the variant of the present disclosure, a microorganism producing L-valine comprising the ketol-acid reductoisomerase variant of the present disclosure or the polynucleotide encoding the same, and a method for producing L-valine using the microorganism of the present disclosure.

### [Background Art]

L-amino acids are the basic building blocks of proteins and are used as important materials for pharmaceutical raw materials, food additives, animal feed, nutritional supplements, insecticides, bactericides, *etc.* In particular, branched-chain amino acids (BCAAs) are a collective term that refers to L-valine, L-leucine, and L-isoleucine, which are essential amino acids. These BCAAs have antioxidant effects and the effect of promoting protein synthesis of muscle cells.

The production of BCAAs using microorganisms is known to be mainly carried out through microorganisms of the genus *Corynebacterium* and biosynthesized from pyruvate through several steps [APPLIED AND ENVIRONMENTAL MICROBIOLOGY, Dec. 2010, p. 8053-8061].

### [Disclosure]

### [Technical Problem]

The production of BCAAs through microorganisms has a problem in that it is not easy to mass produce on an industrial scale.

### [Technical Solution]

An object of the present disclosure is to provide a ketol-acid reductoisomerase variant, wherein the amino acid corresponding to position 87 in the amino acid sequence of SEQ ID NO: 1 is substituted with valine (V) or aspartate (D).

Another object of the present disclosure is to provide a polynucleotide encoding the variant of the present disclosure.

Still another object of the present disclosure is to provide a microorganism comprising the variant of the present disclosure or the polynucleotide encoding the variant of the present disclosure.

Still another object of the present disclosure is to provide a method for producing L-valine, comprising culturing the microorganism of the present disclosure in a medium.

Still another object of the present disclosure is to provide a composition for producing L-valine, comprising: the microorganism of the present disclosure; the medium in which the microorganism is cultured; or a combination of two or more thereof.

### [Advantageous Effects]

High-yield production of L-valine can be achieved by using the variant of the present disclosure.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a ketol-acid reductoisomerase variant comprising a substitution of the amino acid corresponding to position 87 of SEQ ID NO: 1 with another amino acid.

As used herein, the term "ketol-acid reductoisomerase variant" means any polypeptide having ketol-acid reductoisomerase activity or a variant of ketol-acid reductoisomerase comprising a substitution of the amino acid corresponding to position 87 from the N-terminus of SEQ ID NO: 1 with another amino acid.

The variant of the present disclosure may also be described as "a ketol-acid reductoisomerase variant", "a (modified) polypeptide having ketol-acid reductoisomerase activity", or "a IlvC variant".

As used herein, the term "ketol-acid reductoisomerase" refers to an enzyme also referred to as "acetohydroxy acid isomeroreductase", "KARI", and "AHAIR", which is an enzyme involved in the biosynthesis of L-branched-chain amino acids. The ketol-acid reductoisomerase can be classified as EC 1.1.1.86.

Upon examination the biosynthetic pathway of L-branched-chain amino acids, it can be seen that acetohydroxy acid synthase first catalyzes the decarboxylation of pyruvate and the condensation reaction with another pyruvate molecule to produce acetolactate, which is a precursor of valine, or catalyzes the decarboxylation of pyruvate and the condensation reaction with 2-ketobutyrate to produce acetohydroxybutyrate, which is a precursor of isoleucine. Ketol-acid reductoisomerase then uses the thus-produced acetolactate or acetohydroxybutyrate as substrates to proceed with reactions that are capable of producing L-valine, L-leucine, and L-isoleucine.

Specifically, a ketol-acid reductoisomerase can convert 2-aceto-2-hydroxybutyrate to 2,3-dihydroxy-3-methylvalerate, or 2-acetolactate to 2,3-dihydroxyisovalerate.

When the 2,3-dihydroxy-3-methylvalerate undergoes reactions catalyzed by dihydroxy acid dehydratase and transaminase B, L-isoleucine is produced. When the 2,3-dihydroxyisovalerate undergoes a reaction mediated by dihydroxy acid dehydratase, 2-ketoisovalerate is produced. The 2-ketoisovalerate may be converted to L-valine by transaminase B, or to 2-ketoisocaproate, which can subsequently be converted to L-leucine through enzymatic conversion. Therefore, ketol-acid reductoisomerase is an important enzyme in the production of BCAAs including L-valine, L-leucine, and L-isoleucine.

The amino acid sequence of the ketol-acid reductoisomerase of the present disclosure may be an amino acid sequence encoded by the *ilvC* gene and also be referred to as the "IlvC protein". The amino acid sequence of the ketol-acid reductoisomerase of the present disclosure can be obtained from GenBank of NCBI, which is a known database. The ketol-acid reductoisomerase may be a protein comprising the amino acid sequence of SEQ ID NO: 1, but is not limited thereto. In another embodiment, the ketol-acid reductoisomerase may be derived from a microorganism of the genus *Corynebacterium,* for example, *Corynebacterium glutamicum.* Examples may include WP_003854117.1, 6JX2_A, HJE10081.1, WP_059289140.1, WP_060564426.1, WP_006286981.1, WP_096455581.1, WP_066565326.1, WP_015651057.1, WP_053544709.1, WP_006769331.1, BAC18177.1, WP_156227806.1, WP_191733749.1, WP_042621277.1, WP_126119396.1, NLZ56857.1, or WP_015400720.1, but are not limited thereto. Sequences having the same ketol-acid reductoisomerase activity as these amino acid sequences may be included without limitation.

In a specific embodiment, the ketol-acid reductoisomerase of the present disclosure may be a protein comprising the amino acid sequence of SEQ ID NO: 1 or an amino acid sequence having 70%, 75%, 80%, 85%, 90%, 95%, 96%, 97%, 98% or more, or 99% or more homology or identity to the same. Additionally, it is apparent that a protein having an amino acid sequence in which part of the sequence is deleted, modified, substituted, or added is also included within the scope of the protein subject to variation in the present disclosure provided that the amino acid sequence has such homology or identity and exhibits efficacy corresponding to the protein above.

Furthermore, in one embodiment, the ketol-acid reductoisomerase subject to variation of the present disclosure is defined as a protein comprising the amino acid sequence of SEQ ID NO: 1, but it does not exclude the addition of nonsense sequences before or after the amino acid sequence of SEQ ID NO: 1, naturally occurring mutations, or silent mutations thereof. Further, provided that a protein exhibits the same or corresponding activity as the protein consisting of the amino acid sequence of SEQ ID NO: 1, it is apparent to those skilled in the art that this protein corresponds to the ketol-acid reductoisomerase of the present disclosure.

That is, even if the present disclosure discloses "a protein or polypeptide having an amino acid sequence disclosed by a specific SEQ ID NO" or "a protein or polypeptide comprising an amino acid sequence disclosed by a specific SEQ ID NO", it is apparent that a protein having an amino acid sequence in which some sequences are deleted, modified, substituted, or added may also be used in the present disclosure provided that the protein exhibits the same or corresponding activity as the polypeptide consisting of an amino acid sequence of the corresponding SEQ ID NO.

As used herein, the term "homology" or "identity" means the degree of similarity between two given amino acid sequences or nucleotide sequences and may be expressed as a percentage. The terms "homology" and "identity" may often be used interchangeably.

Sequence homology or identity of conserved polynucleotides or polypeptides is determined by standard alignment algorithms, and default gap penalties established by the software used may be used together. Substantially, homologous or identical sequences can generally hybridize with an entire sequence or a part thereof under moderate or highly stringent conditions. It is apparent that hybridization also includes hybridization between polynucleotides containing general codons or codons considering codon degeneracy.

Whether any two polynucleotide or polypeptide sequences exhibit homology, similarity, or identity may be determined, for example, using known computer algorithms such as the "FASTA" program with default parameters as in Pearson et al. (1988) [Proc. Natl. Acad. Sci. USA 85]: 2444. Alternatively, it may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later). (Examples include the GCG program package (Devereux, J., et al., Nucleic Acids Research 12: 387(1984)), BLASTP, BLASTN, and FASTA (Atschul, S. F. et al., J Molec Biol 215]: 403 (1990); Guide to Huge Computers, Martin J. Bishop, ed., Academic Press, San Diego, 1994, and [CARILLO et al.](1988) SIAM J Applied Math 48: 1073).) For example, the sequence homology, similarity, or identity may be determined using the BLAST of the National Center for Biotechnology Information (NCBI) or ClustalW.

The homology, similarity, or identity of polynucleotides or polypeptides may be determined by comparing the sequence information using, for example, the GAP computer program as in Needleman et al. (1970), J Mol Biol. 48:443, for example, as described in Smith and Waterman, Adv. Appl. Math(1981) 2:482,. In summary, the GAP program defines the homology, similarity, or identity of polynucleotides or polypeptides as the ratio of the number of similar aligned symbols (*i.e.*, nucleotides or amino acids) to the total number of symbols in the shorter of the two sequences. The default parameters for the GAP program may include: (1) a unitary comparison matrix (containing a value of 1 for identity and 0 for non-identity), and the weighted comparison matrix (or EDNAFULL (EMBOSS version of NCBI NUC4.4) substitution matrix) from Gribskov et al. (1986) Nucl. Acids Res. 14:6745, as disclosed in Schwartz and Dayhoff, eds., Atlas Of Protein Sequence And Structure, National Biomedical Research Foundation, pp.353-358 (1979); (2) a penalty of 3.0 for each gap and an additional penalty of 0.10 for each symbol within each gap (or gap opening penalty of 10 and gap extension penalty of 0.5); and (3) no penalty for terminal gaps. Therefore, the term "homology" or "identity" used in the present disclosure shows the relevance between sequences.

As used herein, the term "variant" or "modified polypeptide" refers to a protein in which one or more amino acids are different from the recited sequence by conservative substitutions and/or modifications while the functions or properties of the recited sequence are retained.

This variant differs from the identified sequence by several amino acid substitutions, deletions, or additions. Such variant may generally be identified by modifying one or more amino acids in the amino acid sequence of the protein and evaluating the characteristics of the modified protein. That is, the ability of the variant may be increased, remain unchanged, or be decreased compared to that of the native protein. Additionally, some variants may include modified polypeptides with one or more portions, such as N-terminal leader sequences or transmembrane domains, removed. Other variants may include variants of mature proteins in which a portion is removed from the N- and/or C-terminus. The term "variant" or "modified polypeptide" may be used interchangeably with terms such as modification, modified protein, mutant, mutein, divergent, and variant, but are not limited thereto provided that the term is used to mean mutation.

As used herein, the term "conservative substitution" means the substitution of an amino acid with another amino acid having similar structural and/or chemical properties. Conventionally, conservative substitution may have little or no effect on the activity of the produced polypeptide.

The variant may still possess one or more biological activities while having, for example, one or more conservative substitutions. Such amino acid substitutions may generally occur based on the similarities in polarity, charge, solubility, hydrophobicity, hydrophilicity, and/or amphipathic nature of the residues.

As an example of such classification, the amino acids may be classified into: positively charged amino acids (basic), which include arginine, lysine, and histidine; negatively charged amino acids (acidic), which include glutamic acid and aspartate; amino acids with non-polar side chains (non-polar amino acids), which include glycine, alanine, valine, leucine, isoleucine, methionine, phenylalanine, tryptophan, and proline; and amino acids with polar or hydrophilic side chains (polar amino acids), which include serine, threonine, cysteine, tyrosine, asparagine, and glutamine. In another embodiment, the amino acids may be classified into electrically charged amino acids, which include arginine, lysine, histidine, glutamic acid, and aspartate; or uncharged amino acids (also referred to as neutral amino acids), which include glycine, alanine, valine, leucine, isoleucine, methionine, phenylalanine, tryptophan, proline, serine, threonine, cysteine, tyrosine, asparagine, and glutamine. In another embodiment, phenylalanine, tryptophan, and tyrosine may be classified as aromatic amino acids. In another embodiment, valine, leucine, and isoleucine may be classified as branched amino acids. In another embodiment, the 20 amino acids may be classified into five groups by size, in order of the amino acid group having relatively smaller volume: glycine, alanine, and serine; cysteine, proline, threonine, aspartate, and asparagine; valine, histidine, glutamic acid, and glutamine; isoleucine, leucine, methionine, lysine, and arginine; and phenylalanine, tryptophan, and tyrosine, but are not limited thereto.

Additionally, the variant may include deletions or additions of amino acids that have minimal effect on the properties and secondary structure of the polypeptide. For example, the polypeptide may be co-translationally or post-translationally conjugated with a signal (or leader) sequence at the N-terminus of a protein that participates in protein transfer. Further, the polypeptide may be conjugated with another sequence or linker for identification, purification, or synthesis of the polypeptide.

In one embodiment, the variant of the present disclosure may be a ketol-acid reductoisomerase variant in which the amino acid corresponding to position 87 from the N-terminus of SEQ ID NO: 1 is substituted with a different amino acid or a modified polypeptide having ketol-acid reductoisomerase activity among the previously described ketol-acid reductoisomerases.

In any one embodiment of the previously described embodiments, the variant may be capable of increasing the L-valine production compared to the protein before modification, wild-type protein, natural polypeptide, or unmodified polypeptide.

As used herein, "substitution with another amino acid" is not limited as long as the amino acid differs from the amino acid before substitution. Meanwhile, it is apparent that when it is expressed in the present disclosure that "a specific amino acid is substituted", the amino acid is substituted with an amino acid different from the amino acid before substitution even if it is not particularly stated that the amino acid is substituted with a different amino acid.

In any one embodiment of the previously described embodiments, the "another amino acid" may be any amino acid except glutamine (Q). Specifically, the another amino acid may be any one selected from glycine, alanine, valine, leucine, isoleucine, methionine, phenylalanine, tryptophan, proline, serine, threonine, cysteine, tyrosine, asparagine, aspartate, glutamic acid, lysine, arginine, and histidine.

In any one embodiment of the previously described embodiments, the variant may be a ketol-acid reductoisomerase variant in which the amino acid corresponding to position 87 of the amino acid sequence of SEQ ID NO: 1 is substituted with valine (V) or aspartate (D).

As used herein, the term "Nth position" may include the Nth position and the position of the amino acid corresponding to the Nth position. For example, it may include the position of the amino acid corresponding to any amino acid residue in a mature polypeptide disclosed in a specific amino acid sequence. The specific amino acid sequence may be the amino acid sequence of SEQ ID NO: 1.

As used herein, the term "corresponding to" refers to an amino acid residue at the position listed in the polypeptide or an amino acid residue that is similar, identical, or homologous to the residue listed in the polypeptide. Confirmation of an amino acid at the corresponding position may be determination of a specific amino acid in a sequence referencing a particular sequence. The term "corresponding region" used in the present disclosure generally refers to a similar or corresponding position in a related protein or the reference protein.

In the present disclosure, specific numbering may be used for the amino acid residue positions within a protein used in the present disclosure. For example, by aligning the polypeptide sequences of the target protein to be compared and the protein of the present disclosure, it is possible to renumber the positions corresponding to the positions of amino acid residues in the protein of the present disclosure.

For example, any amino acid sequence may be aligned with SEQ ID NO: 1, and based thereon, each amino acid residue of the amino acid sequence may be numbered with reference to the numbered positions of the amino acid residues corresponding to the amino acid residues in SEQ ID NO: 1. For example, sequence alignment algorithms such as disclosed in the present disclosure are capable of identifying the positions of amino acids or the positions of modifications, such as substitutions, insertions, or deletions, by comparing with the query sequence (also referred to as "the reference sequence").

For such alignments, algorithms such as the Needleman-Wunsch algorithm (Needleman and Wunsch, 1970, J. Mol. Biol. 48: 443-453), the Needleman program in the EMBOSS package (EMBOSS: The European Molecular Biology Open Software Suite, Rice et al., 2000, Trends Genet. 16: 276-277), *etc.* may be used, but are not limited thereto, and other sequence alignment programs, pairwise sequence comparison algorithms, *etc.* known in the art may be appropriately used.

In one embodiment, the ketol-acid reductoisomerase variant of the present disclosure may be a variant comprising a sequence in which the amino acid corresponding to position 87 of SEQ ID NO: 1 is substituted with valine (V) or aspartate (D), and having at least 70%, 75%, 80%, 85%, 90%, 95%, 96%, 97%, 98%, or 99% homology or identity with SEQ ID NO: 1. The ketol-acid reductoisomerase variant may be a variant having less than 100% homology or identity with SEQ ID NO: 1.

In any one embodiment of the previously described embodiments, the ketol-acid reductoisomerase variant of the present disclosure may be a polypeptide in which the amino acid corresponding to position 87 of SEQ ID NO: 1 is substituted with valine (V) or aspartate (D), which has at least 70%, 75%, 80%, 85%, 90%, 95%, 96%, 97%, 98%, or 99% homology or identity with SEQ ID NO: 1, and which has ketol-acid reductoisomerase activity.

In any one embodiment of the previously described embodiments, the ketol-acid reductoisomerase variant of the present disclosure may be a polypeptide in which the amino acid corresponding to position 87 of SEQ ID NO: 1 is valine (V) or aspartate (D), which has at least 70%, 75%, 80%, 85%, 90%, 95%, 96%, 97%, 98%, or 99% homology or identity with SEQ ID NO: 1, and which has ketol-acid reductoisomerase activity.

Furthermore, it is apparent that a variant having an amino acid sequence in which part of the sequence has deletions, modifications, substitutions, conservative substitutions, or additions is also included within the scope of the present disclosure provided that the amino acid sequence has such homology or identity and exhibits an effect corresponding to that of the variant of the present disclosure.

In any one embodiment of the previously described embodiments, the variant of the present disclosure may have, comprise, or essentially consist of the amino acid sequence disclosed as SEQ ID NO: 3 or SEQ ID NO: 25.

Even if it is disclosed in the present disclosure as "a protein having an amino acid sequence disclosed as a specific SEQ ID NO", it is apparent that a protein having an amino acid sequence in which part of the sequence has deletions, modifications, substitutions, conservative substitutions, or additions is also included within the scope of the present disclosure provided that the amino acid sequence exhibits the same or corresponding activity as the protein consisting of an amino acid sequence of the corresponding SEQ ID NO. For example, the addition of sequences, naturally occurring mutations, silent mutations thereof, or conservative substitutions before and after the amino acid sequence which do not alter the function of the protein are not excluded provided that this sequence exhibits the same or corresponding activity as the modified protein, and it is apparent that such addition of sequences or mutations are also within the scope of the present disclosure.

In one embodiment of the previously described embodiments, the variant of the present disclosure may comprise a sequence having at least 80%, 90%, 95%, 96%, 97%, 98%, or 99% homology or identity with SEQ ID NO: 3 or SEQ ID NO: 25 or consist of the amino acid sequence. In this variant, the amino acid corresponding to position 87 of SEQ ID NO: 3 or 25 may be valine (V) or aspartate (D).

In any one embodiment of the previously described embodiments, the ketol-acid reductoisomerase variant of the present disclosure may have enhanced ketol-acid reductoisomerase activity, but is not limited thereto.

In any one embodiment of the previously described embodiments, the ketol-acid reductoisomerase variant of the present disclosure may have an activity that increases L-valine producing ability compared to a wild-type, native, or unmodified polypeptide having ketol-acid reductoisomerase activity, but is not limited thereto.

Another aspect of the present disclosure provides a polynucleotide encoding the variant of the present disclosure. The variant is as described in the aspect above.

As used herein, the term "polynucleotide" means a DNA or RNA strand of a certain length or longer, consisting of a polymer of nucleotides in which nucleotide monomers are linked together in a long chain via covalent bonds, and more specifically, a polynucleotide fragment encoding the variant.

The polynucleotide of the present disclosure may undergo various modifications in the coding region within a scope that does not change the amino acid sequence of the variant of the present disclosure, considering codon degeneracy or the preferred codons in the organism to be expressed with the variant of the present disclosure. Therefore, it is apparent that a polynucleotide which may be translated into a polypeptide consisting of the amino acid sequence of the variant of the present disclosure by codon degeneracy or a polypeptide having homology or identity thereto may also be included.

For example, the polynucleotide of the present disclosure may have or comprise a nucleotide sequence having at least 70%, 75%, 80%, 85%, 90%, 95%, 96%, 97%, 98%, or 99% and less than 100% homology or identity with the polynucleotide sequence of SEQ ID NO: 66, or consist or essentially consist of a nucleotide sequence having at least 70%, 75%, 80%, 85%, 90%, 95%, 96%, 97%, 98%, or 99% homology or identity with the polynucleotide sequence of SEQ ID NO: 2, but is not limited thereto. Alternatively, in the polynucleotide sequence having the above homology or identity, the codon encoding the amino acid corresponding to position 87 of SEQ ID NO: 1 may be one of the codons encoding valine (V) or aspartate (D), but is not limited thereto.

Additionally, the polynucleotide of the present disclosure may include, without limitation, probes that can be prepared from known genetic sequences, for example, sequences that are capable of hybridizing with complementary sequences to all or part of the polynucleotide sequence of the present disclosure under stringent conditions. The term "stringent conditions" mean conditions that enable specific hybridizations between polynucleotides. Such conditions are specifically described in literature (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, a list of the conditions may include: hybridization polynucleotides having high homology or identity, that is, polynucleotides having 70% or more, 75% or more, 80% or more, 85% or more, 90% or more, 95% or more, 96% or more, 97% or more, 98% or more, or 99% or more homology or identity, but not hybridizing polynucleotides having lower homology or identity than the above; or the conventional washing conditions for southern hybridization, which corresponds to washing once, specifically two or three times, under a salt concentration and temperature of 60°C, 1XSSC, 0.1% SDS, specifically 60°C, 0.1XSSC, 0.1% SDS, and more specifically 68°C, 0.1XSSC, 0.1% SDS.

Hybridization, even though mismatches between nucleotides are possible depending on the stringency of hybridization, requires two nucleic acids to have complementary sequences. The term "complementary" is used to describe the relationship between nucleotide bases that can hybridize with each other. For example, in DNA, adenine is complementary to thymine, and cytosine is complementary to guanine. Therefore, the polynucleotide of the present disclosure may also include not only nucleic acid sequences that are substantially similar but also isolated nucleic acid fragments that are complementary to the entire sequence.

Specifically, a polynucleotide having homology or identity to the polynucleotide of the present disclosure may be detected using hybridization conditions comprising hybridizing at a Tm value of 55°C and the previously described conditions. Furthermore, the Tm value may be 60°C, 63°C, or 65°C, but is not limited thereto, and may be appropriately adjusted by those skilled in the art according to the purpose.

The appropriate stringency for hybridization of the polynucleotides depends on the lengths and degrees of complementarity of the polynucleotides, and the variables are well known in the art (*e.g.*, J. Sambrook *et al., supra*).

Still another aspect of the present disclosure provides a vector comprising a polynucleotide encoding the variant of the present disclosure. The variant and polynucleotide are as described in the other aspects above.

The vector may be an expression vector for expressing the polynucleotide in microorganisms, but is not limited thereto.

As used herein, the term "vector" may comprise a DNA construct comprising a nucleotide sequence of the polynucleotide encoding a target polypeptide, operably linked to a suitable expression regulatory region (or expression regulatory sequence) such that the target polypeptide can be expressed in an appropriate host. The expression regulatory region may comprise a promotor that can initiate transcription, any operator sequence for regulating such transcription, a sequence encoding a suitable mRNA ribosome binding site, and sequences for regulating the termination of transcription and translation. After transforming into a suitable microorganism, the vector may replicate or function independently of the host genome and may integrate into the genome itself.

The vector used in the present disclosure is not specifically limited, and any vector known in the art may be used. Examples of conventionally used vectors include plasmids, cosmids, viruses, and bacteriophages in their natural or recombinant state. For example, as phage vectors or cosmid vectors, pWE15, M13, MBL3, MBL4, IXII, ASHII, APII, t10, t11, Charon4A, Charon21A, *etc.* may be used, and as plasmid vectors, vectors based on pDZ, pBR, pUC, pBluescriptII, pGEM, pTZ, pCL, pET, *etc.* may be used. Specifically, pDZ, pDC, pDCM2, pACYC177, pACYC184, pCL, pECCG117, pUC19, pBR322, pMW118, pCC1BAC vectors, *etc.* may be used.

In an embodiment, a polynucleotide encoding a target polypeptide may be inserted into a chromosome through a vector for intracellular chromosomal insertion. The insertion of the polynucleotide into the chromosome may be achieved by any known method in the art, such as homologous recombination, but is not limited thereto. A selection marker for confirmation of the chromosomal insertion may be further included. The selection marker is used for selection of the transformed cells with vectors, that is, confirmation of whether the target nucleic acid molecule has been inserted. For the selection marker, markers that provide selectable phenotypes, such as drug resistance, nutrient requirements, resistance to cytotoxic agents, or expression of surface polypeptides, may be used. In an environment treated with a selective agent, only the cells that express the selection markers survive or express other phenotypes, thereby allowing the selection of transformed cells.

In the present disclosure, the term "transformation" means the introduction of a vector comprising a polynucleotide encoding the target polypeptide into a microorganism or a microbe such that the polypeptide encoded by the polynucleotide can be expressed in the microorganism. Transformed polynucleotide include all polynucleotides, regardless of whether they are inserted in the chromosome of a microorganism or located extrachromosomally, as long as the polynucleotides are capable of being expressed within the microorganism. Further, the polynucleotide includes DNA and/or RNA encoding the target polypeptide. The polynucleotide may be introduced in any form as long as it can be introduced into and expressed in a microorganism. For example, the polynucleotide may be introduced into a microorganism in the form of an expression cassette, which is a genetic construct comprising all necessary elements for autonomous expression. The expression cassette may typically comprise a promotor operably linked to the polynucleotide, a transcription termination signal, a ribosome binding site, and a translation termination signal. The expression cassette may be in the form of an expression vector capable of self-replication. Furthermore, the polynucleotide may be introduced into a microorganism as-is and operably linked to a sequence necessary for expression in the microorganism, but is not limited thereto.

Additionally, the term "operably linked" above means that a promoter sequence that initiates and mediates the transcription of a polynucleotide encoding the target variant of the present disclosure and a sequence of the polynucleotide are functionally linked.

As used herein, the term "introduction" means the method of delivering a polynucleotide encoding the variant of the present disclosure or a vector comprising the same to a host cell. Such introduction may be readily performed by conventional methods in the art. Common examples include: CaCl₂ precipitation; Hanahan method, which has increased efficiency using a reducing substance called dimethyl sulfoxide (DMSL) in the CaCl₂ method; electroporation; calcium phosphate precipitation; protoplast fusion; stirring using silicon carbide fibers; transformation using PEG; dextran sulfate, lipofectamine, and desiccation/inhibition-mediated transformation, *etc.* The method for the transformation of the vector is not limited thereto, and transformation or transfection methods conventionally used in the art may be used without limitation.

Still another aspect of the present disclosure provides a microorganism comprising the variant of the present disclosure or the polynucleotide encoding the variant of the present disclosure.

In one embodiment, the microorganism may include a vector including the polynucleotide encoding the variant.

In one embodiment, the microorganism of the present disclosure may be a microorganism having L-valine producing ability.

As used herein, the term "L-valine", which is one of the essential amino acids, means the L-amino acid with the chemical formula (CH₃)₂CHCH(NH₂)COOH, which, along with L-leucine and L-isoleucine, structurally corresponds to the BCAAs.

As used herein, the term "microorganism (or strain)" comprises both the wild-type microorganism, and a naturally or artificially genetically modified microorganism, and refers to a microorganism having a specific mechanism weakened or strengthened due to factors such as the insertion of an exogenous gene, or enhancement or inactivation of activity of an endogenous gene. It may be a microorganism comprising genetic modification for the production of a target polypeptide, protein, or product. As used herein, the terms "microorganism", "strain", and "microbe" have the same meaning and may be used interchangeably without limitation.

As used herein, the term "microorganism producing L-valine" refers to a prokaryotic or eukaryotic microbial strain that is capable of producing L-valine within an organism, and may include both a microorganism in which L-valine producing ability is imparted to a parent strain lacking L-valine producing ability, and a microorganism inherently having L-valine producing ability. The L-valine producing ability may be imparted or enhanced by species improvement.

In one embodiment, the microorganism of the present disclosure may be: a microorganism naturally having ketol-acid reductoisomerase or L-valine producing ability; or a microorganism in which the variant of the present disclosure or a polynucleotide encoding the same (or a vector comprising the polynucleotide) is introduced or L-valine producing ability is imparted to a parent strain lacking ketol-acid reductoisomerase or L-valine producing ability, but is not limited thereto.

In one embodiment, the microorganism of the present disclosure includes both a microorganism comprising the sequence of the ketol-acid reductoisomerase variant of the present disclosure caused by mutation of the gene in the chromosome encoding the ketol-acid reductoisomerase, and/or a microorganism including the ketol-acid reductoisomerase variant of the present disclosure by introducing a vector comprising a polynucleotide encoding the ketol-acid reductoisomerase variant of the present disclosure, but is not limited thereto.

In any one embodiment of the previously described embodiments, the microorganism provided in the present disclosure may be a genetically modified microorganism to express the ketol-acid reductoisomerase of the present disclosure.

As used herein, the term "unmodified microorganism" does not exclude strains comprising mutations that may naturally occur in microorganisms and may be a wild-type strain or natural strain itself, or a strain before its traits are changed by genetic mutation due to natural or artificial factors. For example, the unmodified microorganism may mean a strain in which the ketol-acid reductoisomerase variant described in the present specification is not introduced or has not yet been introduced. The term "unmodified microorganism" may be used interchangeably with "strain before modification", "microorganism before modification", "unvaried strain", "unmodified strain", "unmutated microorganism", or "reference microorganism".

The microorganism having L-valine producing ability of the present disclosure may be: a microorganism comprising any one or more of the variant of the present disclosure, the polynucleotide of the present disclosure, and a vector including the polynucleotide of the present disclosure; a modified microorganism to express the variant of the present disclosure or the polynucleotide of the present disclosure; a microorganism (*e.g.*, a recombinant strain) that expresses the polynucleotide of the present disclosure; or a microorganism (*e.g.*, a recombinant strain) having the activity of the variant of the present disclosure, but is not limited thereto.

In one embodiment, the strain of the present disclosure refers to a cell or microorganism capable of expressing the variant of the present disclosure by the transformation with a vector comprising the polynucleotide of the present disclosure or a polynucleotide encoding the variant of the present disclosure, and the strain of the present disclosure may include all microorganisms that are capable of producing L-valine by comprising the variant of the present disclosure. For example, the microorganism of the present disclosure may be a recombinant strain in which a polynucleotide encoding the variant of the present disclosure is introduced into a natural wild-type microorganism or a microorganism having L-valine producing ability to express a ketol-acid reductoisomerase variant, thereby having increased L-valine producing ability.

The strain with increased L-valine producing ability may be a microorganism with increased L-valine producing ability compared to a natural wild-type microorganism or unmodified ketol-acid reductoisomerase microorganism (for example, a wild-type microorganism expressing ketol-acid reductoisomerase or a microorganism that does not express the variant of the present disclosure), but is not limited thereto.

In one embodiment, the microorganism with increased L-valine producing ability of the present disclosure may be a microorganism with increased L-valine producing ability compared to a microorganism comprising a polypeptide of SEQ ID NO: 1 or a polynucleotide encoding the same, but is not limited thereto.

In one embodiment, the microorganism with increased L-valine producing ability may have an increase of about 1% or more, about 2% or more, about 2.5% or more, or about 3% or more (the upper limit is not particularly limited and may be, e.g., about 200% or less, about 150% or less, about 100% or less, about 50% or less, about 45% or less, about 40% or less, about 35% or less, about 30% or less, about 25% or less, or about 20% or less) compared to the L-valine producing ability of the parent strain before modification or the unmodified microorganism, but is not limited thereto as long as the microorganism with increased L-valine producing ability has a positive value of increase compared to the producing ability of the parent strain before modification or the unmodified microorganism. In another embodiment, the recombinant strain with increased L-valine producing ability may have an increase of about 1.01 times or more, about 1.02 times or more, or about 1.03 times or more (the upper limit is not particularly limited, and may be, e.g., about 10 times or less, about 5 times or less, about 3 times or less, about 2 times or less, or about 1.5 times or less) compared to the L-valine producing ability of the parent strain before modification or the unmodified microorganism, but is not limited thereto.

An example of the parent strain before modification or the unmodified microorganism for comparing the increase in the L-valine producing ability may include a microorganism comprising the polypeptide of SEQ ID NO: 1 or a polynucleotide encoding the same. Other examples may include *Corynebacterium glutamicum* KCCM11201P (US 8465962 B), *Corynebacterium glutamicum* ATCC13869, and *Corynebacterium glutamicum* ATCC14067, but are not limited thereto.

The term "about" refers to a range including values such as ±0.5, ±0.4, ±0.3, ±0.2, ±0.1, *etc.,* and includes all values in the range equivalent or similar to the numerical values following the term "about", but is not limited thereto.

The microorganism of the present disclosure may include all microorganisms capable of expressing the ketol-acid reductoisomerase variant of the present disclosure through various known methods in addition to the introduction of the nucleic acid or vector.

The microorganism of the present disclosure may be a microorganism of the genus *Corynebacterium.*

In one embodiment, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris*, or *Corynebacterium flavescens.* In one embodiment of the previously described embodiments, the microorganism of the present disclosure may be *Corynebacterium glutamicum.*

The microorganism of the genus *Corynebacterium* having L-valine producing ability of the present disclosure includes all of a naturally occurring wild-type microorganism; microorganism of the genus *Corynebacterium* with increased L-valine producing ability by strengthening or weakening the activity of genes related to the L-valine production mechanism; and a microorganism of the genus *Corynebacterium* with increased L-valine producing ability by introducing or strengthening the activity of an exogenous gene.

In one embodiment, the microorganism of the present disclosure may include an acetohydroxy acid synthase modified to have increased L-valine producing ability. In any one embodiment of the previously described embodiments, the microorganism of the present disclosure may include a modified acetohydroxy acid synthase subunit (ilvN). The acetohydroxy acid synthase subunit may include a mutation in which the amino acid at position 42 is substituted with valine (A42V). However, the microorganism of the present disclosure is not limited thereto.

The microorganism of the present disclosure may have enhanced activity of the ketol-acid reductoisomerase variant of the present disclosure.

As used herein, the term "enhancement" of polypeptide activity means that the polypeptide activity is increased compared to its intrinsic activity. The term enhancement may be used interchangeably with terms such as activation, up-regulation, overexpression, increase, *etc.* In particular, activation, enhancement, up-regulation, overexpression, and increase may include both the exhibition of an activity that was not originally present and the exhibition of increased activity compared to the intrinsic activity or activity before modification. The term "intrinsic activity" means the activity of a specific polypeptide that the parent strain or an unmodified microorganism originally possessed before transformation when the traits are transformed by natural or artificial factors. This term may be used interchangeably with "activity before modification". When the activity of a polypeptide is "enhanced", "up-regulated", "overexpressed", or "increased" compared to its intrinsic activity, it means that the activity and/or concentration (expression level) of the specific polypeptide is enhanced compared to that of a specific polypeptide originally possessed by the parent strain or unmodified microorganism.

This enhancement may be achieved by introducing an exogenous polypeptide or enhancing the activity and/or concentration (expression level) of an endogenous polypeptide. Whether the activity of the polypeptide is enhanced may be confirmed from the degree of activity, the expression level of the corresponding polypeptide, or the increase in the amount of products discharged from the polypeptide.

The enhancement of the activity of the polypeptide may be achieved by applying various methods well known in the art and is not limited as long as the activity of the target polypeptide can be enhanced compared to the microorganism before modification. Specifically, the enhancement may be achieved using genetic engineering and/or protein engineering well known to those skilled in the art, which is a common method of molecular biology, but is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be:
1) an increase in the intracellular copy number of the polynucleotide encoding the polypeptide;
2) replacement of the gene expression regulatory region encoding the polypeptide on the chromosome with a sequence having strong activity;
3) modification of the nucleotide sequence encoding the initiation codon or the 5'-UTR region of the gene transcript encoding the polypeptide;
4) modification of the amino acid sequence of the polypeptide to enhance the polypeptide activity;
5) modification of the polynucleotide sequence encoding the polypeptide to enhance the polypeptide activity (for example, modification of the polynucleotide sequence of the polypeptide gene so as to encode a modified polypeptide to enhance the polypeptide activity);
6) introduction of an exogenous polypeptide expressing the polypeptide activity or an exogenous polynucleotide encoding the polypeptide;
7) codon optimization of a polynucleotide encoding the polypeptide;
8) modification or chemical modification of an exposed region selected by analyzing the tertiary structure of the polypeptide; or
9) a combination of two or more selected from 1) to 8), but is not limited thereto.

More specifically:
The 1) increase in the intracellular copy number of the polynucleotide encoding the polypeptide may be achieved by the introduction of a vector that is operably linked to a polynucleotide encoding the corresponding polypeptide, in which the vector is capable of replicating and functioning independently of the host, into a host cell. Alternatively, it may be achieved by inserting one copy, or two or more copies of the polynucleotide encoding the corresponding polypeptide into the chromosome of the host cell. The chromosomal insertion may be performed by introducing a vector capable of inserting the polynucleotide into the chromosome of the host cell, but is not limited thereto. The vector is as described above.

The 2) replacement of the gene expression regulatory region (or expression regulatory sequence) encoding the polypeptide on the chromosome with a sequence having strong activity may be, for example, occurrence of mutation in the sequence including deletion, insertion, non-conservative or conservative substitution, or a combination thereof to further strengthen the activity of the expression regulatory region, or replacement with a sequence having strong activity. The expression regulatory region is not specifically limited thereto but may include a promoter, an operator sequence, a sequence encoding a ribosome binding site, sequences regulating the termination of transcription and translation, *etc.* In an embodiment, it may be replacement of the original promoter with a strong promoter, but is not limited thereto.

Examples of known strong promoters may include the CJ1 to CJ7 promoters (US Patent No. US 7662943 B2), the lac promoter, the trp promoter, the trc promoter, the tac promoter, the lambda phage PR promoter, the PL promoter, the tet promoter, the gapA promoter, the SPL7 promoter, the SPL13 (sm3) promoter (US Patent No. US 10584338 B2), the O2 promoter (US Patent No. US 10273491 B2), the tkt promoter, the yccA promoter, *etc.,* but are not limited thereto.

The 3) modification of the nucleotide sequence encoding the initiation codon or the 5'-UTR region of the gene transcript encoding the polypeptide may be, for example, substitution with a nucleotide sequence encoding a different initiation codon with a higher polypeptide expression rate compared to the intrinsic initiation codon, but is not limited thereto.

The 4) and 5) modification of the amino acid sequence or polynucleotide sequence may be occurrence of mutation such as deletion, insertion, non-conservative or conservative substitution, or a combination thereof in the amino acid sequence of the polypeptide or the polynucleotide sequence encoding the polypeptide to enhance the activity of the polypeptide; or replacement with an amino acid sequence or polynucleotide sequence improved to have stronger activity, or an amino acid sequence or polynucleotide sequence improved to increase in activity, but is not limited thereto. Specifically, the replacement may be performed by inserting a polynucleotide into the chromosome by homologous recombination, but is not limited thereto. The vector used herein may further comprise a selection marker for confirming chromosomal insertion. The selection marker is as described above.

The 6) introduction of an exogenous polynucleotide expressing the activity of the polypeptide may be introduction of an exogenous polynucleotide into a host cell, in which the exogenous polynucleotide encodes a polypeptide exhibiting the same or similar activity as the polypeptide. The exogenous polynucleotide is not limited in its origin or sequence as long as it exhibits the same or similar activity as the polypeptide. The method used for the introduction may be a known transformation method and may be appropriately selected and performed by those skilled in the art, and the activity of the polypeptide may be increased as the introduced polynucleotide is expressed in the host cell thereby producing the polypeptide.

The 7) codon optimization of a polynucleotide encoding the polypeptide may be codon optimization of an endogenous polynucleotide to increase its transcription or translation within a host cell or codon optimization of an exogenous polynucleotide to achieve optimized transcription or translation of the polynucleotide within a host cell.

The 8) modification or chemical modification of an exposed region selected by analyzing the tertiary structure of the polypeptide may be, for example, modification or chemical manipulation of an exposed portion to be modified or chemically manipulated that is selected by comparing the sequence information of the polypeptide to be analyzed with a database storing the sequence information of known proteins, followed by determining the template protein candidates based on the degree of sequence similarity, and confirming the structure based thereon.

Such enhancement of polypeptide activity may be an increase in activity or concentration of the corresponding polypeptide based on the activity or concentration of the polypeptide expressed in a wild-type strain or microorganism strain before modification, or an increase in the amount of the product produced from the corresponding polypeptide, but is not limited thereto.

In the microorganism of the present disclosure, the modification of all or part of a polynucleotide may be induced by: (a) homologous recombination using an intrachromosomal insertion vector within the microorganism or genome editing using an engineered nuclease (*e.g.*, CRISPR-Cas9); and/or (b) light such as ultraviolet or radiation and/or chemical treatment, but the methods are not limited thereto. **The** methods for the modification of all or part of the gene may include a method based on DNA recombination technology. For example, homologous recombination induced by introducing a nucleotide sequence or vector comprising a nucleotide sequence with homology to the target gene into the microorganism may lead to a loss of all or part of the gene. The introduced nucleotide sequence or vector may include a dominant selection marker, but is not limited thereto.

In the microorganism of the present disclosure, the variant, polynucleotide, L-valine, *etc.* are as described in the other aspects above.

Another aspect of the present disclosure provides a method for producing L-valine, comprising culturing the microorganism of the present disclosure in a medium.

Specifically, the method for producing L-valine of the present disclosure may include culturing, in a medium, a microorganism including the variant of the present disclosure, the polynucleotide of the present disclosure, or the vector of the present disclosure, but is not limited thereto.

As used herein, the term "culture" means growth of the microorganism of the present disclosure under appropriately controlled environmental conditions. The culturing procedures of the present disclosure may be performed according to the suitable medium and culture conditions known in the art. Such culturing procedures may easily be adjusted according to the selected strain and used by those skilled in the art. Specifically, the culture may be performed in a batch type, continuous type, and/or fed-batch type, but is not limited thereto.

As used herein, the term "medium" means a mixture of substances containing nutrients required to culture the microorganism of the present disclosure as a main component, and the medium supplies nutrients, growth factors, *etc.,* including water, which are indispensable for survival and development of the microorganism. Specifically, as the medium and other culture conditions used in the culture of the microorganism of the present disclosure, any one medium may be used without particular limitation provided that the medium is commonly used for the culture of microorganisms while the microorganism of the present disclosure may be cultured in a common medium containing suitable carbon sources, nitrogen sources, phosphorus sources, inorganic compounds, amino acids and/or vitamins, while controlling the temperature, pH, *etc.* under aerobic conditions.

For example, the culture medium for the strain of the genus *Corynebacterium* may be found in literature ["Manual of Methods for General Bacteriology" by the American Society for Bacteriology (Washington D.C., USA, 1981)].

In the present disclosure, the carbon sources may include carbohydrates such as glucose, saccharose, lactose, fructose, sucrose, maltose, *etc.;* sugar alcohols such as mannitol, sorbitol, *etc.;* organic acids such as pyruvic acid, lactic acid, citric acid, *etc.;* and amino acids such as glutamic acid, methionine, lysine, *etc.* Further, natural organic nutrients such as starch hydrolysate, molasses, blackstrap molasses, rice bran, cassava, sugarcane molasses, and corn steep liquor may be used. Specifically, carbohydrates such as glucose and sterilized pretreated molasses (*i.e.*, molasses converted to reducing sugar) may be used, and appropriate amounts of other carbon sources may be used in various manners without limitation. These carbon sources may be used alone or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, ammonium nitrate, *etc.;* and organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, defatted soybean cake or decomposition products thereof, *etc.* may be used. These nitrogen sources may be used alone or in combination of two or more thereof, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, *etc.* may be used. Additionally, amino acids, vitamins and/or suitable precursors may be included. These constituents or precursors may be added to the medium batchwise or continuously, but the methods are not limited thereto.

Further, during the culture of the microorganism of the present disclosure, the pH of the medium may be adjusted by adding compounds such as ammonium hydroxide, potassium hydroxide, ammonia, phosphoric acid, sulfuric acid, *etc.* to the medium in an appropriate manner. Additionally, foaming may be suppressed during the culture using an antifoaming agent such as fatty acid polyglycol ester. Furthermore, oxygen or oxygen-containing gas may be injected into the medium to maintain the aerobic state of the medium, or gas may not be injected or nitrogen, hydrogen, or carbon dioxide gas may be injected to maintain the anaerobic and microaerobic states, but the methods are not limited thereto.

In the culture of the present disclosure, the culture temperature may be maintained at 20°C to 45°C, specifically, at 25°C to 40°C, and the strain may be cultured for about 10 hours to 160 hours, but the culture conditions are not limited thereto.

The L-valine produced by the culture of the present disclosure may be secreted into the medium or remain in the cells.

The method for producing L-valine of the present disclosure may further comprise preparing the microorganism of the present disclosure, preparing a medium for culturing the microbial strain, or a combination thereof (in any order), for example, prior to the culture.

The method for producing L-valine of the present disclosure may further comprise recovering L-valine from the medium according to the culture (the medium subjected to the culture) or from the microorganism of the present disclosure. The recovery may further be included after the culture.

The recovery may be to collect L-valine using a suitable method known in the art according to the culture method for the microorganism of the present disclosure, for example, a batch, continuous, or fed-batch culture method. For example, centrifugation, filtration, treatment with a crystallized protein precipitant (salting out), extraction, sonication, ultrafiltration, dialysis, various forms of chromatography such as molecular sieve chromatography (gel filtration), adsorption chromatography, ionexchange chromatography, and affinity chromatography, HPLC, or a combination thereof may be used. L-valine may be recovered from the medium or microorganism using a suitable method known in the art.

Further, the method for producing L-valine of the present disclosure may further include purification. The purification may be performed using a suitable method known in the art. In one embodiment, when the method for producing L-valine of the present disclosure includes both the recovery and purification, the recovery and purification may be performed continuously or discontinuously in any order, or simultaneously or integrated into a single step, but the methods are not limited thereto.

In the method for the present disclosure, the variant, polynucleotide, vector, microorganism, L-valine *etc.* are as described in the other aspects above.

Another aspect of the present disclosure provides a composition for producing L-valine, the composition comprising the microorganism of the present disclosure, the medium in which the microorganism is cultured; or a combination of two or more thereof.

The composition of the present disclosure may further comprise any suitable excipients that are commonly used in compositions for producing amino acids. Such excipients may be, for example, preservatives, wetting agents, dispersing agents, suspending agents, buffers, stabilizers, isotonic agents, *etc.,* but are not limited thereto.

In the composition of the present disclosure, the variant, polynucleotide, vector, microorganism, L-valine, *etc.* are as described in the other aspects above.

Still another aspect of the present disclosure provides a use of the ketol-acid reductoisomerase variant of the present disclosure in producing L-valine. The variant is as described in the other aspects above.

### [Mode for Carrying Out the Invention]

Hereinafter, the present disclosure will be described in more detail with reference to Examples. However, these Examples are only for illustrating the present disclosure, and the scope of the present disclosure is not intended to be limited by these Examples. Meanwhile, technical matters not described in this specification can be sufficiently understood and readily implemented by those skilled in the art or in related technical fields.

### Example 1-1: Induction of artificial mutations through UV irradiation

In order to select a mutant strain with increased valine producing ability, the valine-producing strain *Corynebacterium glutamicum* KCCM11201P (US 8465962 B) was plated onto a nutrient medium containing agar and cultured at 30°C for 36 hours. Hundreds of thus-obtained colonies were subjected to UV irradiation at room temperature to induce random mutations in the genome of the strain.

### Example 1-2. Evaluation of fermentation potency and selection of mutant strains

To select a mutant strain with increased L-valine producing ability compared to the parent strain *Corynebacterium glutamicum* KCCM11201P, fermentation potency experiments were conducted on the strains induced with random mutations. Each colony was subcultured in a nutrient medium, and then each strain was inoculated into a 250 mL corner-baffled flask containing 25 mL of production medium, and cultured at 30°C with shaking at 200 rpm for 72 hours. Subsequently, the concentration of L-valine was analyzed using HPLC. The L-valine concentrations analyzed are shown in Table 1 below.

### [Nutrient Medium (pH 7.2)]

glucose 10 g, meat extract 5 g, polypeptone 10 g, sodium chloride 2.5 g, yeast extract 5 g, agar 20 g, and urea 2 g (per 1 liter of distilled water)

### [Production Medium (pH 7.0)]

glucose 100 g, ammonium sulfate 40 g, soy protein 2.5 g, corn steep solids 5 g, urea 3 g, dipotassium phosphate 1 g, magnesium sulfate heptahydrate 0.5 g, biotin 100 µg, thiamine-HCl 1 mg, calcium pantothenate 2 mg, nicotinamide 3 mg, and calcium carbonate 30 g (per 1 liter of distilled water)

**[Table 1]**

| | Strain Name | L-valine (g/L) |
|---|---|---|
| Control group | KCCM11201P | 2.9 |
| Experimental group | C1 | 3.0 |
| | C2 | 2.5 |
| | C3 | 2.3 |
| | C4 | 2.6 |
| | C5 | 2.3 |
| | C6 | 1.9 |
| | C7 | 1.2 |
| | C8 | 3.8 |
| | C9 | 3.0 |
| | C10 | 3.1 |
| | C11 | 1.8 |
| | C12 | 4.3 |
| | C13 | 3.5 |
| | C14 | 2.9 |
| | C15 | 1.6 |
| | C16 | 2.2 |
| | C17 | 2.7 |
| | C18 | 2.5 |
| | C19 | 2.7 |
| | C20 | 3.4 |
| | C21 | 2.3 |

With reference to Table 1, C12 strain, which has the greatest increase in valine production compared to the control group KCCM11201P strain, was selected.

### Example 2. Confirmation of mutations through gene sequencing

The major genes of the C12 strain were sequenced and compared with those of the KCCM11201P strain and the wild-type *Corynebacterium glutamicum* ATCC14067 strain. As a result, it was confirmed that this strain with increased valine producing ability included nucleotide sequence mutations at specific positions in the open reading frame (ORF) region of the *ilvC* gene. Specifically, the C12 strain, which showed the greatest increase in valine production, was confirmed to have three mutations introduced to the nucleotide sequence located at 259-261 bp downstream from the initiation codon of the *ilvC* gene, resulting in a form where the original CAG (SEQ ID NO: 66) was changed to GTT (SEQ ID NO: 2) and the 87^{th} amino acid, glutamine, was substituted with valine (SEQ ID NO: 3).

The analysis of the mutation region in SEQ ID NO: 2 confirmed that it affects the effector binding domain of the valine biosynthesis enzyme. Thus, it was predicted that the activity of the protein would be enhanced. In the examples herein, it was confirmed whether the application of the Q87V mutation inserted at a specific position within the ORF of the *ilvC* gene affects the ability of a microorganism of the genus *Corynebacterium* to produce valine, a branched-chain amino acid. Further, it was confirmed whether the substitution of the 87^{th} amino acid, glutamine, with another amino acid (except valine) affects the ability of a microorganism of the genus *Corynebacterium* to produce valine, a branched-chain amino acid.

### Example 3. Production of KCCM11201P strain with ilvC mutation and confirmation of L-valine production

### Example 3-1. Production of Corynebacterium glutamicum KCCM11201P strain with ilvC mutation and evaluation of L-valine producing ability

In order to insert the *ilvC* (Q87V) mutant represented by SEQ ID NO: 2 into *glutamicum* KCCM11201P, a vector including the target mutation was prepared. Specifically, the genomic DNA of the C12 strain was extracted using a G-spin Total DNA extraction mini kit (Intron, Cat. No. 17045) according to the protocol provided in the kit, and PCR was performed using the genomic DNA as a template. The PCR was performed under the following conditions: denaturation at 94°C for 5 minutes; 25 cycles of denaturation at 94°C for 30 seconds, annealing at 55°C for 30 seconds, and polymerization at 72°C for 150 seconds; and polymerization at 72°C for 7 minutes. A 1,010 bp PCR product (hereinafter referred to as "mutation-introduced fragment 1") was obtained using SEQ ID NO: 4 and SEQ ID NO: 5.

The obtained mutation-introduced fragment 1 was ligated into a pDCM2 vector (Korean Patent Application Publication No. 10-2020-0136813) treated with Smal (New England Biolabs, Beverly, MA) using the Infusion Cloning Kit (Takara Bio Inc., Otsu, Japan) and then transformed into *Escherichia coli* DH5α. After transforming the thus-prepared gene into *Escherichia coli* DH5α, the strain was selected on LB medium containing kanamycin, and DNA was extracted therefrom using the DNA-spin Plasmid DNA Purification Kit (iNtRON) to prepare a pDCM2-ilvC(Q87V) vector comprising the mutation-introduced fragment 1.

**[Table 2]**

| Primer | Nucleotide sequence |
|---|---|
| SEQ ID NO: 4 | CCCGGG GCACTGCTTGATGTGATGGAACCAT |
| SEQ ID NO: 5 | CCCGGG GTCAACGATGAGCTTGAGCTCGT |

The pDCM2-ilvC(Q87V) vector was transformed into *Corynebacterium glutamicum* KCCM11201P by homologous recombination on the chromosome (van der Rest et al., Appl Microbiol Biotechnol 52:541-545, 1999). The resulting strain, in which the vector was inserted into the chromosome by homologous recombination, was selected on medium containing 25 mg/L of kanamycin. Then the *Corynebacterium glutamicum* transgenic strain for which secondary recombination was completed was subjected to PCR using SEQ ID NO: 4 and SEQ ID NO: 5 to confirm a strain in which glutamine at the amino acid position 87 of SEQ ID NO: 1 within the ORF of the *ilvC* gene on the chromosome is substituted with valine. This recombinant strain was named *Corynebacterium glutamicum* KCCM11201P::*ilvC*(Q87V)*.* To compare the valine producing ability of the valine-producing strain *Corynebacterium glutamicum* KCCM11201P and the recombinant strain *Corynebacterium glutamicum* KCCM11201P::*ilvC*(Q87V), a flask evaluation was performed. Each strain was subcultured in nutrient medium, inoculated in a 250 mL corner-baffled flask containing 25 ml of production medium, and cultured at 30°C with shaking at 200 rpm for 72 hours. Then, the L-valine concentrations were analyzed using HPLC. The L-valine concentrations analyzed are shown in Table 3 below.

### [Nutrient Medium (pH 7.2)]

glucose 10 g, meat extract 5 g, polypeptone 10 g, sodium chloride 2.5 g, yeast extract 5 g, agar 20 g, and urea 2 g (per 1 liter of distilled water)

### [Production Medium (pH 7.0)]

glucose 100 g, ammonium sulfate 40 g, soy protein 2.5 g, corn steep solids 5 g, urea 3 g, dipotassium phosphate 1 g, magnesium sulfate heptahydrate 0.5 g, biotin 100 µg, thiamine-HCl 1 mg, calcium pantothenate 2 mg, nicotinamide 3 mg, and calcium carbonate 30 g (per 1 liter of distilled water)

**[Table 3]**

| L-valine producing ability of KCCM11201P and KCCM11201P::*ilvC*(Q87V) | | | | |
|---|---|---|---|---|
| Strain | L-valine (g/L) | | | |
| | Batch 1 | Batch 2 | Batch 3 | Mean |
| KCCM11201P | 2.8 | 2.7 | 2.8 | 2.7 |
| KCCM11201P::*ilvC*(Q87V) | 3.1 | 3.2 | 3.3 | 3.2 |

As a result, the L-valine producing ability of the KCCM11201P::*ilvC*(Q87V) strain increased by 16% compared to KCCM11201P.

### Example 3-2: Production of Corynebacterium glutamicum CJ7V strain with ilvC mutation and evaluation of L-valine producing ability

To confirm whether other *Corynebacterium glutamicum* strains also exhibit increased L-valine producing ability, a strain with enhanced L-valine producing ability was prepared by introducing a single mutation [ilvN(A42V); Biotechnology and Bioprocess Engineering, June 2014, Volume 19, Issue 3, pp 456-467] into the wild-type *Corynebacterium glutamicum* ATCC14067 strain.

Specifically, the genomic DNA of the ATCC14067 strain, which is the wild-type strain of *Corynebacterium glutamicum,* was extracted using a G-spin Total DNA extraction mini kit (Intron, Cat. No. 17045) according to the protocol provided in the kit. PCR was performed using the genomic DNA as a template. In order to prepare a vector that introduces an A42V mutation into the *ilvN* gene, gene fragments (A, B) were obtained using a primer pair of SEQ ID NO: 6 and SEQ ID NO: 7, and a primer pair of SEQ ID NO: 8 and SEQ ID NO: 9, respectively. The PCR was performed using the following conditions: denaturation at 94°C for 5 minutes; 25 cycles of denaturation at 94°C for 30 seconds, annealing at 55°C for 30 seconds, and polymerization at 72°C for 60 seconds; and polymerization at 72°C for 7 minutes.

As a result, polynucleotides of 528 bp and 509 bp were obtained for fragments A and B, respectively. Overlapping PCR was performed using the two fragments as templates together with SEQ ID NO: 6 and SEQ ID NO: 9 to obtain a 1,010 bp PCR product (hereinafter referred to as "mutation-introduced fragment 2").

The obtained mutation-introduced fragment 2 was treated with the restriction enzyme Smal (New England Biolabs, Beverly, MA), then ligated with the pDCM2 vector, which was also treated with the same restriction enzyme, using T4 ligase (New England Biolabs, Beverly, MA). The prepared gene was transformed into *Escherichia coli* DH5α, then selected on LB medium containing kanamycin. The DNA was obtained using the DNA-spin Plasmid DNA Purification Kit (iNtRON). The vector targeting the introduction of a A42V mutation of the *ilvN* gene was named pDCM2-ilvN(A42V).

**[Table 4]**

| Primer | Nucleotide sequence |
|---|---|
| SEQ ID NO: 6 | cggggatcccccgggAGGACGGTACTCAAATACTAAACTTC |
| SEQ ID NO: 7 | TGCCGAGTGTTTCGGTCTTTACAGACACGAGGGACACG |
| SEQ ID NO: 8 | TGTCTGTAAAGACCGAAACACTCGGCATCAA |
| SEQ ID NO: 9 | cggggatcccccgggGACAACTACATTATTATTATACCACA |

Subsequently, the pDCM2-ilvN(A42V) vector was transformed into the wild-type *Corynebacterium glutamicum* ATCC14067 strain by homologous recombination on the chromosome (van der Rest et al., Appl Microbiol Biotechnol 52:541-545, 1999). The resulting strain, in which the vector was inserted into the chromosome by homologous recombination, was selected on medium containing 25 mg/L of kanamycin. Gene fragments of the *Corynebacterium glutamicum* transgenic strain, for which the second recombination was completed, were amplified through PCR using SEQ ID NO: 6 and SEQ ID NO: 9, and then the mutant insertion strain was confirmed through gene sequence analysis. This recombinant strain was named *Corynebacterium glutamicum* CJ7V. Finally, using *Corynebacterium glutamicum* CJ7V, a strain transformed with the vector was prepared as in Example 3-1, and the resulting strain was named *Corynebacterium glutamicum CJ7V*::*ilvC*(Q87V). To compare the L-valine producing ability of the thus-prepared strain, the strain was cultured and the L-valine concentration was analyzed as in Example 3-1, and the L-valine concentrations analyzed are shown in Table 5 below.

**[Table 5]**

| L-valine producing ability of CJ7V and CJ7V::*ilvC*(Q87V) | | | | |
|---|---|---|---|---|
| Strain | L-valine (g/L) | | | |
| | Batch 1 | Batch 2 | Batch 3 | Mean |
| CJ7V | 2.2 | 2.1 | 2.2 | 2.2 |
| CJ7V::*ilvC*(Q87V) | 2.6 | 2.5 | 2.6 | 2.6 |

As a result, it was confirmed that the L-valine producing ability of CJ7V::*ilvC*(Q87V) is increased by 18% compared to CJ7V.

### Example 3-3: Production of Corynebacterium glutamicum CJ8V strain with ilvC mutation and evaluation of L-valine producing ability

In order to confirm whether other *Corynebacterium glutamicum* strains that produce L-valine also have the effect of increased L-valine producing ability , a strain with enhanced L-valine producing ability was prepared by introducing a single mutation [ilvN(A42V); Biotechnology and Bioprocess Engineering, June 2014, Volume 19, Issue 3, pp 456-467] into the wild-type strain *Corynebacterium glutamicum* ATCC13869 (KR 10-1947945 B1).

Specifically, the pDCM2-ilvN(A42V) vector prepared in Example 3-2 was transformed into the wild-type *Corynebacterium glutamicum* ATCC13869 strain, thereby inducing homologous recombination on the chromosome (van der Rest et al., Appl Microbiol Biotechnol 52:541-545, 1999). The resulting strain, in which the vector was inserted into the chromosome by homologous recombination, was selected on medium containing 25 mg/L of kanamycin. The selected *Corynebacterium glutamicum* transgenic strain was subjected to PCR using a primer pair of SEQ ID NO: 10 and SEQ ID NO: 11 sequences to amplify the gene fragment, and the proper introduction of mutation was confirmed through gene sequence analysis. This recombinant strain was named *Corynebacterium glutamicum* CJ8V. The sequences of the primers used in this example are listed in Table 6 below.

**[Table 6]**

| Primer | Nucleotide sequence |
|---|---|
| SEQ ID NO: 10 | CCGCGTCACCAAAGCGGA |
| SEQ ID NO: 11 | TTAGATCTTGGCCGGAGCCA |

Finally, using the *Corynebacterium glutamicum* CJ8V strain, a strain transformed with the vector was prepared in the same manner as in Example 3-1, which was named *Corynebacterium glutamicum* CJ8V::*ilvC*(Q87V). To compare the L-valine producing ability of the prepared strain, the strain was cultured and its L-valine concentration was analyzed in the same manner as in Example 3-1. The L-valine concentrations analyzed are as shown in Table 7 below.

**[Table 7]**

| L-valine producing ability of CJ8V and CJ8V::*ilvC*(Q87V) | | | | |
|---|---|---|---|---|
| Strain | L-valine (g/L) | | | |
| | Batch 1 | Batch 2 | Batch 3 | Mean |
| CJ8V | 1.9 | 1.8 | 1.9 | 1.9 |
| CJ8V*::ilvC(*Q87V*)* | 2.3 | 2.3 | 2.3 | 2.3 |

As a result, CJ8V::*ilvC*(Q87V) exhibited a 21% increase in L-valine producing ability compared to CJ8V.

### Example 4. Production of KCCM11201P strain with Q87X mutation and confirmation of L-valine production

In order to mutate glutamine, the amino acid at position 87 of *ilvC,* with another amino acid other than valine, the pDCM2-ilvC(Q87V) vector used in Example 3-1 was used as the template for site-directed mutagenesis. The site-directed mutagenesis was performed with the method described below.

**[Table 8]**

| PCR composition for site-direction mutagenesis | |
|---|---|
| | Unit (µL) |
| 10X pfu-X Buffer | 5 |
| 10mM dNTP Mix | 1 |
| pfu-X Polymerase | 1 |
| Mutagenic forward primer (5 pmol) | 2 |
| Mutagenic reverse primer (5 pmol) | 2 |
| pDCM2-ilvC(Q87V) (template DNA, 200 ng/µL) | 1 |
| dH₂O | 38 |
| Total | 50 |

**[Table 9]**

| PCR cycle for site-direction mutagenesis | | |
|---|---|---|
| Cycle | Temperature | Time |
| 1 | 95°C | 1 min |
| 18 | 95°C | 50 sec |
| | 60°C | 50 sec |
| | 68°C | 9 min |
| 1 | 68°C | 7 min |

To substitute the 87^{th} amino acid of *ilvC* (*i.e.,* glutamine) with any of the other amino acids other than valine, such as alanine (A, SEQ ID NO: 12), valine (V, SEQ ID NO: 3), isoleucine (I, SEQ ID NO: 13), glycine (G, SEQ ID NO: 14), phenylalanine (F, SEQ ID NO: 15), methionine (M, SEQ ID NO: 16), serine (S, SEQ ID NO: 17), proline (P, SEQ ID NO: 18), threonine (T, SEQ ID NO: 19), tyrosine (Y, SEQ ID NO: 20), histidine (H, SEQ ID NO: 21), glutamine (Q, SEQ ID NO: 22), asparagine (N, SEQ ID NO: 23), lysine (K, SEQ ID NO: 24), aspartate (D, SEQ ID NO: 25), cysteine (C, SEQ ID NO: 26), tryptophan (W, SEQ ID NO: 27), arginine (R, SEQ ID NO: 28), and glutamate (E, SEQ ID NO: 29), PCR mixtures as shown in [Table 8] were prepared using each of the mutagenic primer sets described in [Table 10], and PCR was performed using the cycle described in [Table 9]. After completion of each PCR, it was confirmed that the pDCM2_ilvC mutant plasmids obtained by ligation using the Infusion Cloning Kit (Takara Bio Inc., Otsu, Japan) and transforming into *Escherichia coli* DH5α were replaced with each mutation listed in [Table 10] through sequencing.

**[Table 10]**

| Mutagenic primer sets for preparing plasmids with amino acid mutation at position 87 in amino acid sequence of *ilvC* | | |
|---|---|---|
| Mutant ilvC plasmid | SEQ ID NO | Sequence (5'-3') |
| pDCM2_ilvC(Q87A) | 30 | CAGACACCTCCgcgGCAGAAATCTTCACCAAC |
| | 31 | AAGATTTCTGCcgcGGAGGTGTCTGGAGCCAG |
| pDCM2_ilvC(Q87I) | 32 | CAGACACCTCCattGCAGAAATCTTCACCAAC |
| | 33 | AAGATTTCTGCaatGGAGGTGTCTGGAGCCAG |
| pDCM2_ilvC(Q87G) | 34 | CAGACACCTCCggaGCAGAAATCTTCACCAAC |
| | 35 | AAGATTTCTGCtccGGAGGTGTCTGGAGCCAG |
| pDCM2_ilvC(Q87F) | 36 | CAGACACCTCCttcGCAGAAATCTTCACCAAC |
| | 37 | AAGATTTCTGCgaaGGAGGTGTCTGGAGCCAG |
| pDCM2_ilvC(Q87M) | 38 | CAGACACCTCCatgGCAGAAATCTTCACCAAC |
| | 39 | AAGATTTCTGCcatGGAGGTGTCTGGAGCCAG |
| pDCM2_ilvC(Q87S) | 40 | CAGACACCTCCtctGCAGAAATCTTCACCAAC |
| | 41 | AAGATTTCTGCagaGGAGGTGTCTGGAGCCAG |
| pDCM2_ilvC(Q87P) | 42 | CAGACACCTCCcctGCAGAAATCTTCACCAAC |
| | 43 | AAGATTTCTGCaggGGAGGTGTCTGGAGCCAG |
| pDCM2_ilvC(Q87T) | 44 | CAGACACCTCCaccGCAGAAATCTTCACCAAC |
| | 45 | AAGATTTCTGCggtGGAGGTGTCTGGAGCCAG |
| pDCM2_ilvC(Q87Y) | 46 | CAGACACCTCCtatGCAGAAATCTTCACCAAC |
| | 47 | AAGATTTCTGCataGGAGGTGTCTGGAGCCAG |
| pDCM2_ilvC(Q87H) | 48 | CAGACACCTCCcatGCAGAAATCTTCACCAAC |
| | 49 | AAGATTTCTGCatgGGAGGTGTCTGGAGCCAG |
| pDCM2_ilvC(Q87Q) | 50 | CAGACACCTCCcagGCAGAAATCTTCACCAAC |
| | 51 | AAGATTTCTGCctgGGAGGTGTCTGGAGCCAG |
| pDCM2_ilvC(Q87N) | 52 | CAGACACCTCCaatGCAGAAATCTTCACCAAC |
| | 53 | AAGATTTCTGCattGGAGGTGTCTGGAGCCAG |
| pDCM2_ilvC(Q87K) | 54 | CAGACACCTCCaagGCAGAAATCTTCACCAAC |
| | 55 | AAGATTTCTGCcttGGAGGTGTCTGGAGCCAG |
| pDCM2_ilvC(Q87D) | 56 | CAGACACCTCCgatGCAGAAATCTTCACCAAC |
| | 57 | AAGATTTCTGCatcGGAGGTGTCTGGAGCCAG |
| pDCM2_ilvC(Q87C) | 58 | CAGACACCTCCtgtGCAGAAATCTTCACCAAC |
| | 59 | AAGATTTCTGCacaGGAGGTGTCTGGAGCCAG |
| pDCM2_ilvC(Q87W) | 60 | CAGACACCTCCtggGCAGAAATCTTCACCAAC |
| | 61 | AAGATTTCTGCccaGGAGGTGTCTGGAGCCAG |
| pDCM2_ilvC(Q87R) | 62 | CAGACACCTCCagaGCAGAAATCTTCACCAAC |
| | 63 | AAGATTTCTGCtctGGAGGTGTCTGGAGCCAG |
| pDCM2_ilvC(Q87E) | 64 | CAGACACCTCCgaaGCAGAAATCTTCACCAAC |
| | 65 | AAGATTTCTGCttcGGAGGTGTCTGGAGCCAG |

Each of the pDCM2_ilvC(Q87A), pDCM2_ilvC(Q87I), pDCM2_ilvC(Q87G), pDCM2_ilvC(Q87F), pDCM2_ilvC(Q87M), pDCM2_ilvC(Q87S), pDCM2_ilvC(Q87P), (Q87T), pDCM2_ilvC (Q87Y), pDCM2_ilvC(Q87H), pDCM2_ilvC(Q87Q), pDCM2_ilvC(Q87N), pDCM2_ilvC(Q87K), pDCM2_ilvC(Q87D), pDCM2_ilvC(Q87C), pDCM2_ilvC(Q87W), pDCM2_ilvC(Q87R), and pDCM2_ilvC(Q87E) vectors, which is prepared as in [Table 10] above, was transformed into KCCM11201P by electroporation, and 18 strains with the mutant *ilvC* gene inserted on the chromosome were obtained through a second crossover process. The genetic manipulation was confirmed by the PCR method using primers SEQ ID NO: 4 and SEQ ID NO: 5 that can amplify each of the external regions of the upstream and downstream regions of each homologous recombinant, and genome sequencing.

The thus-obtained transgenic strains were named as follows: KCCM11201P::ilvC(Q87A), KCCM11201P::ilvC(Q87I), KCCM11201P::ilvC(Q87G), KCCM11201P::ilvC(Q87F), KCCM11201P::ilvC(Q87M), KCCM11201P::ilvC(Q87S), KCCM11201P::ilvC(Q87P), KCCM11201P::ilvC(Q87T), KCCM11201P::ilvC(Q87Y), KCCM11201P::ilvC(Q87H), KCCM11201P::ilvC(Q87Q), KCCM11201P::ilvC(Q87N), KCCM11201P::ilvC(Q87K), KCCM11201P::ilvC(Q87D), KCCM11201P::ilvC(Q87C), KCCM11201P::ilvC(Q87W), KCCM11201P::ilvC(Q87R), and KCCM11201P::ilvC(Q87E).

In order to confirm the valine production of the KCCM11201P::ilvC(Q87A), KCCM11201P::ilvC(Q87I), KCCM11201P::ilvC(Q87G), KCCM11201P::ilvC(Q87F), KCCM11201P::ilvC(Q87M), KCCM11201P::ilvC(Q87S), KCCM11201P::ilvC(Q87P), KCCM11201P::ilvC(Q87T), KCCM11201P::ilvC(Q87Y), KCCM11201P::ilvC(Q87H), KCCM11201P::ilvC(Q87Q), KCCM11201P::ilvC(Q87N), KCCM11201P::ilvC(Q87K), KCCM11201P::ilvC(Q87D), KCCM11201P::ilvC(Q87C), KCCM11201P::ilvC(Q87W), KCCM11201P::ilvC(Q87R), and KCCM11201 P::ilvC(Q87E) strains along with that of the KCCM11201P::ilvC(Q87V) strain, these strains were cultured and the L-valine concentrations were analyzed in the same manner as in Example 3-1. The L-valine concentrations analyzed are shown in Table 11 below.

**[Table 11]**

| L-valine producing ability of strains with mutant replacement of 87^{th} amino acid in amino acid sequence of *ilvC* | | |
|---|---|---|
| | Strain | L-valine (g/L) |
| Control group | KCCM11201P | 2.8 |
| Experimental group | KCCM11201P::ilvC(Q87V) | 3.3 |
| | KCCM11201P::ilvC(Q87A) | 2.8 |
| | KCCM11201P::ilvC(Q87I) | 2.8 |
| | KCCM11201P::ilvC(Q87G) | 2.8 |
| | KCCM11201P::ilvC(Q87F) | 2.3 |
| | KCCM11201P::ilvC(Q87M) | 2.4 |
| | KCCM11201P::ilvC(Q87S) | 2.8 |
| | KCCM11201P::ilvC(Q87P) | 2.4 |
| | KCCM11201P::ilvC(Q87T) | 2.8 |
| | KCCM11201P::ilvC(Q87Y) | 2.5 |
| | KCCM11201P::ilvC(Q87H) | 2.8 |
| | KCCM11201P::ilvC(Q87Q) | 2.8 |
| | KCCM11201P::ilvC(Q87N) | 2.8 |
| | KCCM11201P::ilvC(Q87K) | 2.8 |
| | KCCM11201P::ilvC(Q87D) | 2.9 |
| | KCCM11201P::ilvC(Q87C) | 2.5 |
| | KCCM11201P::ilvC(Q87W) | 2.5 |
| | KCCM11201P::ilvC(Q87R) | 2.7 |
| | KCCM11201P::ilvC(Q87E) | 2.8 |

As a result, the L-valine producing ability of the KCCM11201P::ilvC(Q87V) strain was increased by 16% compared to that of KCCM11201P, and the L-valine producing ability of the KCCM11201P:ilvC(Q87D) strain was also increased compared to that of KCCM11201P. The strains where other mutations were introduced showed L-valine-producing ability equivalent to that of KCCM11201P or the effect was insignificant.

From the above, it was confirmed that the variant of the present disclosure can increase L-valine production of microorganisms.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A ketol-acid reductoisomerase variant, wherein the amino acid corresponding to 87th position in the amino acid sequence of SEQ ID NO: 1 is substituted with valine (V) or aspartate (D).

2. The variant of claim 1, wherein the variant comprises SEQ ID NO: 3, SEQ ID NO: 25, or an amino acid sequence having sequence identity of 80% or more thereto.

3. A polynucleotide encoding the variant of claim 1 or 2.

4. A microorganism comprising a ketol acid reductoisomerase variant, wherein the amino acid corresponding to position 87 in the amino acid sequence of SEQ ID NO: 1 is substituted with valine (V) or aspartate (D), or a polynucleotide encoding the variant.

5. The microorganism of claim 4, wherein the microorganism is a microorganism of the genus *Corynebacterium.*

6. The microorganism of claim 5, wherein the microorganism of the genus *Corynebacterium* is *Corynebacterium glutamicum.*

7. A method for producing L-valine, comprising culturing a microorganism comprising a ketol acid reductoisomerase variant, wherein the amino acid corresponding to position 87 in the amino acid sequence of SEQ ID NO: 1 is substituted with valine (V) or aspartate (D), or a polynucleotide encoding the variant in a medium.

8. The method for producing L-valine of claim 7, wherein the method further comprises recovering L-valine.

9. A composition for producing L-valine, comprising a microorganism comprising a ketol acid reductoisomerase variant, wherein the amino acid corresponding to position 87 in the amino acid sequence of SEQ ID NO: 1 is substituted with valine (V) or aspartate (D), or a polynucleotide encoding the variant; a medium in which the microorganism is cultured; or a combination of two or more thereof.
